# EUROPÄISCHE PATENTSCHRIFT

(11) **EP 0 624 362 B1**
(45) Veröffentlichungstag und Bekanntmachung des Hinweises auf die Patenterteilung: **06.03.1996**
(21) Anmeldenummer: 94102174.3
(22) Anmeldetag: 12.02.1994
(51) Int. Cl.: A61K 7/13

(54) **Verfahren zum oxidativen Färben bzw. Nachfärben von menschlichen Haaren**
Method for oxidative dying or recolouring of human hair
Méthode pour teinture oxidative ou renouveler la teinture des cheveux humains

(30) Priorität: 30.04.1993 DE 4314253
(43) Veröffentlichungstag der Anmeldung: 17.11.1994
(73) Patentinhaber: GOLDWELL GmbH, D-64297 Darmstadt (DE)
(72) Erfinder: Tennigkeit, Jürgen, Dr., D-64342 Seeheim (DE); Lorenz, Heribert, D-64372 Gross-Bieberau (DE); Much, Guenter, D-64297 Darmstadt (DE)

(56) Entgegenhaltungen:
- EP-A- 0 465 340
- DE-A- 3 530 270
- DE-A- 3 628 397
- DE-A- 3 628 398
- DE-A- 3 801 606
- GB-A- 2 205 111

## Beschreibung

Die vorliegende Erfindung betrifft ein neues Verfahren zum oxidativen Färben bzw. Nachfärben von menschlichen Kopfhaaren, das nicht nur haarschonender arbeitet als bisher bekannte und übliche Verfahren, sondern auch eine verbesserte Deckkraft der Farben bewirkt und es darüberhinaus auch gestattet, auf Basis einer relativ geringen Auswahl verschiedener Farbmischungen eine Vielzahl gewünschter Farbnuancen zu erzeugen.

Die nach wie vor übliche Art der Haarfärbung ist die Färbung mit Oxidationsfarbstoffen, die unmittelbar vor der Anwendung mit Oxidationsmittel-Zusammensetzungen, insbesondere auf Basis von verdünntem Wasserstoffperoxid, vermischt und auf das Haar aufgetragen werden.

Der pH-Wert dieser gebrauchsfertigen Zusammensetzungen liegt dabei in der Regel im alkalischen Bereich, insbesondere bei pH-Werten zwischen etwa 9 und etwa 10.

Durch diese alkalische Behandlung wird das Haar, insbesondere bei häufiger Wiederholung des Färbevorgangs, in seiner Struktur geschädigt, vor allem, wenn es sich um bereits vorgeschädigtes Haar handelt. Dieses poröse Haar weist nach der Färbung auch eine schlechte Farbbeständigkeit auf.

Es wurde bereits vorgeschlagen, diese Nachteile der bisher üblichen Haarfärbung dadurch zu überwinden, daß, in Abkehr von der oben beschriebenen Praxis, anstelle der alkalisch eingestellten Zusammensetzung aus Oxidationsfarbstoff und Oxidationsmittel eine analoge Zusammensetzung eingesetzt wird, deren pH-Wert im schwach sauren Bereich zwischen etwa 5,9 und 6,9 liegt.

Derartige Zusammensetzungen, die z.B. in den DE-OSen Nr. 35 30 270, 36 28 397 und 36 28 398 beschrieben sind, haben sich in der Tat als wesentlich weniger haarschädigend als alkalische Produkte erwiesen.

In manchen Fällen, d.h., bei Einstellung spezieller Farbnuancen, läßt allerdings die Deckkraft der mit diesen verbesserten Zusammensetzungen erzielten Haarfärbungen etwas zu wünschen übrig.

Die Erfindung hat sich die Aufgabe gestellt, diese Nachteile zu überwinden.

Die Lösung dieser Aufgabe besteht in der Entwicklung eines neuen Verfahrens zum oxidativen Färben bzw. Nachfärben menschlicher Haare, das aus den folgenden Verfahrensschritten besteht:
a) Aufbringung einer alkalisch eingestellten, mindestens eine Entwickler- und mindestens eine Kupplersubstanz sowie ein Oxidationsmittel enthaltenden Oxidationsfarbstoffmischung vorwiegend auf den der Kopfhaut benachbarten Teil des menschlichen Haares, d.h., den "Haaransatz", wobei die Mischung bereits auf den bei der Gesamtfärbung erwünschten Farbton eingestellt ist;
b) Spülen und Shampoonieren des Haares nach erfolgter Farbstoffeinwirkung;
c) Aufbringung einer sauer eingestellten, mindestens eine Entwickler-und mindestens eine Kupplersubstanz sowie ein Oxidationsmittel enthaltenden Oxidationsfarbstoffmischung auf das gesamte Haar, und
d) Spülen des Haares nach erfolgter Farbstoffeinwirkung.

Durch die Anwendung dieses Verfahrens wird nicht nur eine schonende Färbung des Haares mit einer ausgezeichneten Farbdeckkraft erreicht, sondern es ist auch möglich, durch Verwendung spezieller, auf den erwünschten Endton eingestellter Farbmischungen bereits in der ersten Färbestufe den durch die zweite Färbungsstufe dann schließlich zu erreichenden, von der Verbraucherin ausgewählten Farbton durch Anwendung geeigneter Farbvormischungen zu applizieren, ohne daß es dazu der Vorhaltung aller Einzelzusammensetzungen für diesen Farbton bedarf.

Dies stellt sowohl für den Haarfarbenhersteller als auch den Friseur eine wesentliche Vereinfachung dar.

Gemäß einer bevorzugten Ausführungsform der Erfindung ist die Einwirkungsdauer des Färbemittels in der zweiten Behandlungsstufe mit insgesamt etwa 10 bis etwa 30, vorzugsweise etwa 15 bis etwa 20 Minuten kürzer als in der ersten Behandlungsstufe, wo sie bei etwa 20 bis 50, vorzugsweise bei etwa 30 bis 45 Minuten liegt.

Diese Zeitspannen können durch Applikation von Wärme, etwa 30 bis 50° C, vorzugsweise etwa 40°C, um etwa ein Viertel bis zur Hälfte reduziert werden.

Falls es sich bei der Haarfärbung um eine Erstfärbung handelt, ist es zweckmäßig, die alkalische Farbbehandlung über die gesamte Länge des Haares vorzunehmen, wobei aber auch hier die Ansatzfärbung im Vordergrund steht.

Sowohl die in der ersten Behandlungsstufe vorwiegend am sogenannten "Haaransatz", d.h., dem der Kopfhaut am nächsten liegenden Teil des Haarschaftes, zum Einsatz gelangenden, alkalisch eingestellten Oxidationsfarbstoffmischungen als auch die in der zweiten Behandlungsstufe verwendeten, sauer eingestellten Oxidationsfarbstoffmischungen, die jeweils mindestens je eine Entwickler- und je eine Kupplersubstanz sowie ein Oxidationsmittel enthalten, sind an sich bereits bekannt.

Bezüglich der alkalisch eingestellten Mischungen, deren pH-Wert zwischen etwa 8 und 10,5, vorzugsweise 9 und 10, insbesondere bei etwa 9,5 liegt, wird hierzu auf den Stand der Technik, z.B. auf die Monographie von K. Schrader, "Grundlagen und Rezepturen der Kosmetika", 2. Aufl., S 784-804 (1989), verwiesen; die dort beschriebenen Produkte sind im Rahmen des erfindungsgemäßen Verfahrens ebenso einsetzbar wie die aus dem umfangreichen Stand der Technik bekannten weiteren Entwickler- und Kupplersubstanzen sowie Nuanceure.

Gleiches gilt hinsichtlich der sauer, d.h., auf einen pH-Wert von etwa 5 bis 7, insbesondere etwa 5,5 bis 6,9, vorzugsweise 6,7 bis 6,8 eingestellten Farbstoffmischungen, die beispielsweise aus den bereits genannten DE-OSen bekannt sind.

Als Oxidationsmittel wird vor allem eine verdünnte Wasserstoffperoxid-Lösung, -Emulsion oder ein -Gel eingesetzt, möglich, aber weniger üblich ist auch die Verwendung weiterer Peroxide wie Harnstoffperoxid, Melaminperoxid, etc. in entsprechenden stöchiometrischen Mengen.

Die Zusammensetzungen können als Lösungen, Cremes, Pasten, Gele, Aerosole, etc. Verwendung finden.

In den folgenden Beispielen wird die Erfindung illustriert.

### Beispiel 1

20 ml einer alkalischen Oxidationsfarbstoffmischung aus:
- 16,0 (Gew.-%): Cetylstearylalkohol
- 2,8: Stearinmonoethanolamid
- 2,8: Cocosmonoethanolamid
- 1,5: Propylenglykolmono/distearat
- 3,0: Ölsäure
- 2,5: 1,2-Propylenglykol
- 0,5: Natriumlaurylsulfat
- 0,5: Komplexbildner
- 0,5: Ammoniumchlorid
- 1,0: Natriumsulfit
- 10,0: Monoethanolamin
- 2,5: Eiweißhydrolysat
- 0,2: Parfum
- 2,6: p-Toluylendiaminsulfat
- 1,0: Resorcin
- 0,2: 3-Aminophenol
- 0,03: o-Chlor-p-phenylendiamin
- 0,03: 4-Hydroxyethyl-2-aminoanisolsulfat
- ad 100,00: Wasser
werden mit
5ml eines Gemisches aus:
- 16,0: (Gew.-%) Cetylstearylalkohol
- 2,8: Stearinmonoethanolamid
- 2,8: Cocosmonoethanolamid
- 1,5: Propylenglykolmono/distearat
- 3,0: Ölsäure
- 2,5: 1,2-Propylenglykol
- 0,5: Natriumlaurylsulfat
- 0,5: Komplexbildner
- 0,5: Ammoniumchlorid
- 1,0: Natriumsulfit
- 8,7: Monoethanolamin
- 2,5: Eiweißhydrolysat
- 0,2: Parfum
- 0,6: p-Toluylendiaminsulfat
- 0,4: p-Amino-o-kresol
- 0,5: 4-Aminophenol
- ad 100,00: Wasser
und
50 ml 6%iger wäßriger H₂0₂-Lösung vermischt.

Das erhaltene Gemisch, das einen pH-Wert von 9,5 aufweist, wird auf den Haaransatz einer Probandin aufgebracht und gut eingekämmt. Nach 45 Minuten wird das Haar gespült, shampooniert und in seiner Gesamtheit mit einer Zusammensetzung, die durch Vermischen von 40 ml 2%-iger wäßriger Wasserstoffperoxid-Lösung und
20 ml einer Farbzusammensetzung aus
- 9,0: (Gew.-%) Cetylstearylalkohol
- 2,0: Eiweißhydrolysat
- 1,0: Ölsäure
- 1,6: Stearinmonoethanolamid
- 2,6: Cocosmonoethanolamid
- 1,5: Natriumlaurylsulfat
- 0,3: Natriumsulfit
- 0,3: Natriumhydroxid
- 0,3: Ammoniumchlorid
- 0,5: Hydroxyethylcellulose
- 0,1: Mangandioxid
- 0,2: Komplexbildner
- 0,3: Parfum
- 0,6: HC Red No. 3
- 0,4: p-Toluylendiaminsulfat
- 0,3: 4-Aminophenol
- 0,3: 4-Amino-2-hydroxytoluol
- 0,1: Natriumpicramat
- 0,06: 3-Aminophenol
- 0,03: Resorcin
- ad 100,00: Wasser
erhalten wurde und einen pH-Wert von 6,8 aufweist, behandelt.

Nach 20-minütiger Einwirkung wird gespült, shampooniert und getrocknet.

Es wird eine gleichmäßige, ausdruckstarke Färbung des Haares mit einem Teakton erhalten.

### Beispiel 2

20 ml einer alkalischen Oxidationsfarbstoffmischung aus:
- 16,0: (Gew.-%) Cetylstearylalkohol
- 2,8: Stearinmonoethanolamid
- 2,8: Cocosmonoethanolamid
- 1,5: Propylenglykolmono/distearat
- 3,0: Ölsäure
- 2,5: 1,2-Propylenglykol
- 0,5: Natriumlaurylsulfat
- 0,5: Komplexbildner
- 0,5: Ammoniumchlorid
- 1,0: Natriumsulfit
- 12,2: Monoethanolamin
- 2,5: Eiweißhydrolysat
- 0,2: Parfum
- 1,9: p-Toluylendiaminsulfat
- 0,75: Resorcin
- 0,1: 3-Aminophenol
- 0,07: 2-Methylresorcin
- ad 100,00: Wasser
werden mit
5ml einer alkalischen Oxidationsfarbstoffmischung
- 16,0: (Gew.-%) Cetylstearylalkohol
- 2,8: Stearinmonoethanolamid
- 2,8: Cocosmonoethanolamid
- 1,5: Propylenglykolmono/distearat
- 3,0: Ölsäure
- 2,5: 1,2-Propylenglykol
- 0,5: Natriumlaurylsulfat
- 0,5: Komplexbildner
- 0,5: Ammoniumchlorid
- 0,8: Natriumsulfit
- 8,7: Monoethanolamin
- 2,5: Eiweißhydrolysat
- 0,2: Parfum
- 0,5: 4-Aminophenol
- 0,1: p-Amino-o-kresol
- 0,1: 2-Methylresorcin
- ad 100,00: Wasser
und
50 ml 6%igen Wasserstoffperoxid-Lösung vermischt.

Das erhaltene Gemisch, das einen pH-Wert von 9,5 aufweist, wird auf den Haaransatz einer Kundin aufgebracht und gut eingekämmt. Nach 35 Minuten wird das Haar gespült, shampooniert und in seiner Gesamtheit mit einer Zusammensetzung, die durch Vermischen von
40 ml 2%-iger wäßriger Wasserstoffperoxid-Lösung und
20 ml einer Farbzusammensetzung aus
- 9,0: (Gew.-%) Cetylstearylalkohol
- 2,0: Eiweißhydrolysat
- 1,6: Stearinmonoethanolamid
- 2,2: Cocosmonoethanolamid
- 1,5: Natriumlaurylsulfat
- 1,0: Ölsäure
- 0,5: Hydroxyethylcellulose
- 0,3: Natriumsulfit
- 0,3: Natriumhydroxid
- 0,2: Komplexbildner
- 0,3: Ammoniumchlorid
- 0,2: Parfum
- 0,1: Mangandioxid
- 0,6: p-Toluylendiaminsulfat
- 0,1: 4-Aminophenol
- 0,1: 4-Amino-2-hydroxytoluol
- 0,1: 3-Aminophenol
- 0,2: Natriumpicramat
- 0,05: Resorcin
- ad 100,00: Wasser
erhalten wurde und einen pH-Wert von 6,8 aufweist, behandelt.

Nach 15-minütiger Einwirkung wird gespült, shampooniert und getrocknet.

Es wird eine gleichmäßige, ausdruckstarke goldbraune Färbung des Haares erhalten.

### Beispiel 3

20 ml einer alkalischen Oxidationsfarbstoffmischung aus:
- 16,0: (Gew.-%) Cetylstearylalkohol
- 2,8: Stearinmonoethanolamid
- 2,8: Cocosmonoethanolamid
- 1,5: Propylenglykolmono/distearat
- 3,0: Ölsäure
- 2,5: 1,2-Proylenglykol
- 0,5: Natriumlaurylsulfat
- 0,5: Komplexbildner
- 0,5: Ammoniumchlorid
- 0,8: Natriumsulfit
- 11,5: Monoethanolamin
- 2,5: Eiweißhydrolysat
- 0,2: Parfum
- 1,0: p-Toluylendiaminsulfat
- 0,4: Resorcin
- 0,05: 3-Aminophenol
- 0,1: 2-Methylresorcin
- ad 100,00: Wasser
werden mit
5ml einer Oxidationsfarbstoffmischung aus
- 16,0: (Gew.-%) Cetylstearylalkohol
- 2,8: Stearinmonoethanolamid
- 2,8: Cocosmonoethanolamid
- 1,5: Propylenglykolmono/distearat
- 3,0: Ölsäure
- 2,5: 1,2-Propylenglykol
- 0,5: Natriumlaurylsulfat
- 0,5: Komplexbildner
- 0,5: Ammoniumchlorid
- 0,8: Natriumsulfit
- 9,0: Monoethanolamin
- 2,5: Eiweißhydrolysat
- 0,2: Parfum
- 0,25: p-Toluylendiaminsulfat
- 0,08: Resorcin
- 0,02: 3-Aminophenol
- 0,06: 0-Chlor-p-phenylendiamin
- 0,09: 4-Hydroxyethyl-2-aminoanisolsulfat
- ad 100,00: Wasser
und
50 ml einer 6%igen H₂0₂-Lösung vermischt.

Das erhaltene Gemisch, das einen pH-Wert von 9,5 aufweist, wird auf den Haaransatz einer Kundin aufgebracht und gut eingekämmt. Nach 30 minütiger Einwirkung bei etwa 45°C wird das Haar gespült, shampooniert und in seiner Gesamtheit mit einer Zusammensetzung, die durch Vermischen von
40 ml 2%-iger wäßriger Wasserstoffperoxid-Lösung und
20 ml einer Farbzusammensetzung aus
- 9,0 (Gew.-%): Cetylstearylalkohol
- 2,0: Eiweißhydrolysat
- 1,6: Stearinmonoethanolamid
- 2,2: Cocosmonoethanolamid
- 1,5: Natriumlaurylsulfat
- 1,0: Ölsäure
- 0,5: Hydroxyethylcellulose
- 0,3: Natriumsulfit
- 0,3: Natriumhydroxid
- 0,2: Komplexbildner
- 0,2: Parfum
- 0,3: Ammoniumchlorid
- 0,12: Mangandioxid
- 0,45: p-Toluylendiaminsulfat
- 0,5: 4-Aminophenol
- 0,01: 4-Amino-2-hydroxytoluol
- 0,02: Resorcin
- 0,03: 3-Aminophenol
- 0,005: m-Phenylendiamin
- ad 100,00: Wasser
erhalten wurde und einen pH-Wert von 6,8 aufweist, behandelt.

Nach 15-minütiger Einwirkung bei etwa 40°C wird gespült, shampooniert und getrocknet.

Es wird eine gleichmäßige, ausdruckstarke natürliche Mittelaschblondfärbung des Haares erhalten.

### Beispiel 4

25 ml einer alkalischen Oxidationsfarbstoffmischung aus:
- 16,0 (Gew.-%): Cetylstearylalkohol
- 2,8: Stearinmonoethanolamid
- 2,8: Cocosmonoethanolamid
- 1,5: Propylenglykolmono/distearat
- 3,0: Ölsäure
- 2,5: 1,2-Proylenglykol
- 0,5: Natriumlaurylsulfat
- 0,5: Komplexbildner
- 0,5: Ammoniumchlorid
- 1,0: Natriumsulfit
- 11,5: Monoethanolamin
- 2,5: Eiweißhydrolysat
- 0,2: Parfum
- 0,8: p-Toluylendiaminsulfat
- 0,3: Resorcin
- 0,04: 3-Aminophenol
- 0,1: 2-Methylresorcin
- ad 100,00: Wasser
werden mit 50ml einer 6%igen wäßrigen H₂0₂-Lösung verrührt.

Das erhaltene Gemisch, das einen pH-Wert von 9,5 aufweist, wird auf den Haaransatz einer Kundin aufgebracht und gut eingekämmt. Nach 40 Minuten wird das Haar gespült, shampooniert und in seiner Gesamtheit mit einer Zusammensetzung, die durch Vermischen von
40 ml 2%-iger wäßriger Wasserstoff-Peroxid-Lösung und
20 ml einer Farbzusammensetzung aus
- 9,0 (Gew.-%): Cetylstearylalkohol
- 2,0: Eiweißhydrolysat
- 1,6: Stearinmonoethanolamid
- 2,2: Cocosmonoethanolamid
- 1,5: Natriumlaurylsulfat
- 1,0: Ölsäure
- 0,5: Hydroxyethylcellulose
- 0,3: Natriumsulfit
- 0,2: Natriumhydroxid
- 0,2: Komplexbildner
- 0,3: Ammoniumchlorid
- 0,1: Calciumchlorid
- 0,2: Parfum
- 0,3: p-Toluylendiaminsulfat
- 0,07: 4-Aminophenol
- 0,01: 4-Amino-2-hydroxytoluol
- 0,04: Natriumpicramat
- 0,04: 3-Aminophenol
- 0,03: Resorcin
- 0,01: HC Yellow No. 5
- ad 100,00: Wasser
erhalten wurde und einen pH-Wert von 6,7 aufweist, behandelt.

Nach 20-minütiger Einwirkung bei etwa 40°C wird gespült, shampooniert und getrocknet.

Es wird eine gleichmäßige, ausdruckstarke sandblonde Färbung des Haares erhalten.

## Patentansprüche

1. Verfahren zum oxidativen Färben bzw. Nachfärben von menschlichen Haaren, gekennzeichnet durch die Kombination folgender Verfahrensschritte:
a) Aufbringung einer alkalisch eingestellten, mindestens eine Entwickler- und mindestens eine Kupplersubstanz sowie ein Oxidationsmittel enthaltenden Oxidationsfarbstoffmischung vorwiegend auf den der Kopfhaut benachbarten Teil des menschlichen Haares, wobei die Mischung auf den bei der Gesamtfärbung erwünschten Farbton eingestellt ist;
b) Spülen und Shampoonieren des Haares nach erfolgter Farbstoffeinwirkung;
c) Aufbringung einer sauer eingestellten, mindestens eine Entwickler-und mindestens eine Kupplersubstanz sowie ein Oxidationsmittel enthaltenden Oxidationsfarbstoffmischung auf das gesamte Haar, und
d) Spülen des Haares nach erfolgter Farbstoffeinwirkung.

2. Verfahren nach Anspruch 1, dadurch gekennzeichnet, daß die Einwirkungsdauer des Färbemittels in der zweiten Behandlungsstufe kürzer ist als in der ersten Behandlungsstufe.

3. Verfahren nach Anspruch 1 oder 2, dadurch gekennzeichnet, daß die Farbstoffeinwirkung bei etwa 30 bis 50°C erfolgt.

4. Verfahren nach einem oder mehreren der Ansprüche 1 bis 3, dadurch gekennzeichnet, daß die in der ersten Behandlungsstufe zum Einsatz gelangende Oxidationsfarbstoffmischung durch Vermischen zweier Standard-Farbstoffzusammensetzungen bereits auf den nach Abschluß des gesamten Färbeverfahrens zu erreichenden Farbton eingestellt ist.

## Claims

1. Process for oxidative dyeing and re-dyeing of human hair, characterized by the combination of the following sequential steps:
a) Application of an alkaline oxidation hair dye composition comprising at least one developing and at least one coupling substance as well as an oxidizing agent, mainly to the hair region next to the scalp, whereby the composition is adjusted to the hair shade desired for the total hair dyeing result;
b) rinsing and shampooing of the hair after completed processing of the dyeing composition;
c) application of an acidic oxidation hair dye composition comprising at least one developing substance and at least one coupling substance as well as an oxidizing agent, to the total hair; and
d) rinsing of the hair after complete processing of the hair dye.

2. Process according to claim 1, characterized in that the processing time of the dyeing substance of the second step of the dyeing procedure is shorter than that of the first step.

3. Process according to claim 1 or 2, characterized in that the processing action of the hair dye is performed at about 30 to 50°C.

4. Process according to one or more of claims 1 to 3, characterized in that the oxidation hair dye composition used in the first step is already adjusted to the shade to be realized after the completion of the total hair dyeing procedure by admixture of two standard hair dye compositions.

## Revendications

1. Procédé de teinture ou de surteinture oxydante des cheveux humains, caractérisé par la combinaison des étapes suivantes :
a) application d'un mélange colorant oxydant alcalin contenant au moins un révélateur et au moins un coupleur ainsi qu'un oxydant, principalement sur la partie des cheveux humains proche du cuir chevelu, le mélange étant ajusté de sorte à procurer le ton souhaite pour la totalité de la coloration ;
b) rinçage et shampooinage des cheveux après l'action de coloration ;
c) application d'un mélange colorant oxydant acide contenant au moins un révélateur et au moins un coupleur ainsi qu'un oxydant sur la totalité des cheveux, et
d) rinçage des cheveux après l'action de coloration.

2. Procédé selon la revendication 1, caractérisé en ce que la durée d'action du colorant dans la deuxième étape de traitement est plus courte que dans la première.

3. Procédé selon la revendication 1 ou 2, caractérisé en ce que l'action du colorant s'effectue à environ 30 à 50°C.

4. Procédé selon l'une ou plusieurs des revendications 1 à 3, caractérisé en ce que le mélange colorant oxydant utilisé lors de la première étape du traitement est déjà ajusté de sorte à procurer le ton souhaité après mise en oeuvre de la totalité du procédé de teinture, par mélange de deux compositions de colorant standard.
